# EUROPÄISCHE PATENTSCHRIFT

(11) **EP 0 393 425 B1**
(45) Veröffentlichungstag und Bekanntmachung des Hinweises auf die Patenterteilung: **15.09.1993**
(21) Anmeldenummer: 90106481.6
(22) Anmeldetag: 05.04.1990
(51) Int. Cl.: A61F 2/46, A61F 2/36

(54) **Einzementierbares Oberschenkelteil einer Hüftgelenk-Endoprothese**
Femoral part of a hip joint endoprothesis implantable with cement
Partie fémorale implantable avec ciment d'une endoprothèse pour l'articulation de la hanche

(30) Priorität: 15.04.1989 DE 3912465
(43) Veröffentlichungstag der Anmeldung: 24.10.1990
(73) Patentinhaber: Bristol-Myers Squibb Company, New York, N.Y. 10154 (US)
(72) Erfinder: Skripitz, Walter, Dr.med., D-5400 Koblenz (DE); Schelhas, Klaus-Dieter, D-2800 Bremen 66 (DE)
(74) Vertreter: Eisenführ, Speiser & Partner

(56) Entgegenhaltungen:
- DE-A- 3 247 726
- DE-A- 3 829 361
- DE-B- 2 641 770
- DE-B- 2 754 352
- GB-A- 2 082 072
- US-A- 4 274 163

## Beschreibung

Die Erfindung betrifft ein einzementierbares Oberschenkelteil einer Hüftgelenk-Endoprothese, mit einem sich zum unteren Ende hin verjüngenden Schaft, und mit einem flanschartigen Stützkragen auf dem Schaft.

Derartige Oberschenkelteile sind z.B. aus der DE-OS 32 47 726 bekannt, bei denen der Schaft in Längsrichtung dem Knochenhohlraum des natürlichen Oberschenkelknochens angepaßt ist. Aufgrund der Formanpassung an den natürlichen Knochenhohlraum vergrößert sich der Schaftquerschnitt zum Stützkragen hin kontinuierlich und nimmt unterhalb des Stützkragens eine ovale, etwa elliptische Form ein, deren Längsachse etwa in einer medialen/lateralen Richtung verläuft, während die Querachse etwa medial/dorsal ausgerichtet ist. Beim Implantieren derartiger Oberschenkelteile wird der zuvor entsprechend vorbereitete Knochenhohlraum mit Knochenzement gefüllt, anschließend wird das Oberschenkelteil in den frischen Knochenzement eingedrückt. Zur Erzielung eines festen, über eine lange Benutzungszeit stabilen und lockerungsfreien Prothesen-/Knochenverbundes soll dabei der Schaft über seine gesamte Länge und den gesamten Umfang mit einer ausreichend dicken, relativ homogenen Zementschicht umgeben sein, um einerseits den Schaft formschlüssig im Zement, und andererseits den Zement formschlüssig in dem angrenzenden Knochengewebe zu verankern. In der Praxis hat es sich jedoch gezeigt, daß beim Einsetzen des Oberschenkelteils in das Zementbett der frische Zement im proximalen Abschnitt des Schaftes, also unterhalb des Stützkragens, insbesondere im medialen Bereich weitgehend verdrängt wird, so daß sich in diesem Bereich nur eine dünne oder gar keine Zementschicht ausbildet. Ist jedoch die Zementschicht in diesen für die Krafteinleitung wesentlichen Bereich zu dünn, so kann es durch die ständige, aufgrund der Gehbewegung im wesentlichen periodische Belastung des Oberschenkelteils zu einer stärkeren Relativbewegung zwischen Oberschenkelteil und Knochen, und damit auch zu einer Relativbewegung zwischen Schaft und der Zementhülle kommen, wodurch Teile der dünnen Zementschicht brechen und auf diese Weise eine Lockerung des gesamten Oberschenkelteils bewirken können. Außerdem besteht bei derartigen bekannten Oberschenkelteilen die Gefahr, daß sich zwischen der Resektionskante des natürlichen Patientenfemurs und dem Stützkragen ebenfalls keine ausreichende, inhomogene oder teilweise sogar unterbrochene Zementschicht ausbildet, so daß ein direkter Kontakt zwischen Knochen und im Stützkragen mit den damit verbunden ungünstigen Auswirkungen nicht sicher vermieden werden kann.

Aufgabe der Erfindung ist es daher, das einzementierbare Oberschenkelteil der eingangs genannten Art derart weiterzubilden, daß auch im medialen Bereich eine ausreichende Verankerung des Schaftes und zwischen Stützkragen und Patientenfemur eine durchgängige, gleichmäßige Zementschicht verwirklicht wird.

Diese Aufgabe wird bei dem Oberschenkelteil der eingangs genannten Art erfindungsgemäß gelöst durch einen um den Schaft umlaufenden, einen Abstand zwischen der Schaftoberfläche und der Knochenwand festlegenden, abtrennbaren elastischen Distanzring an der Unterseite des Stützkragens, und eine von oben durch den Stützkragen hindurchlaufende Bohrung, welche im oberen Schaftabschnitt aus dem Oberschenkelteil austritt.

Alternativ wird diese Aufgabe bei dem Oberschenkelteil der eingangs genannten Art erfindungsgemäß gelöst durch einen um den Schaft umlaufenden, einen Abstand zwischen der Schaftoberfläche udn der Knochenwand festlegenden, abtrennbaren elastischen Distanzring, der an der Unterseite des Stützkragens anliegt, und eine von oben durch den Stützkragen hindurchlaufende Bohrung, die sich im oberen Schaftabschnitt in mehrere aus dem Schaft austretende Zweigbohrungen verzweigt.

Die Vorteile der Erfindung liegen insbesondere darin, daß der elastische Distanzring beim Einsetzen des Oberschenkelteils eine Zentrierung relativ zu der Wandung des Patientenfemurs ermöglicht, und daß anschließend durch die Bohrung und ggf. die Zweigbohrungen der gesamte, bzw. der noch fehlende Teil des Knochenzements zwischen Oberschenkelteil und Patientenfemur hineingepreßt wird. Aufgrund der Vorfixierung des Oberschenkelteils mittels des Distanzringes kann der Zwischenraum zwischen Oberschenkelteil und Knochenwand definiert festgelegt werden, und der nachträglich in diesen Zwischenraum eingepreßte Knochenzement füllt diesen Zwischenraum aus, ohne daß sich dabei das Oberschenkelteil im Knochenhohlraum verlagert und dabei inhomogene Zementbereiche erzeugt. Nach dem Einpressen einer ausreichenden Menge an Knochenzement wird der Distanzring z.B. an einer entsprechenden Lasche vom Oberschenkelteil abgezogen, so daß nun - beim weiteren Nachpressen von Knochenzement - auch der vom Distanzring erzeugte Freiraum von Knochenzement ausgefüllt wird, so daß der unmittelbare Kontakt zwischen dem metallischen Stützkragen und dem Patientenfemur zuverlässig durch eine homogendicke Zementschicht verhindert wird. Erforderlichenfalls kann das Oberschenkelteil abschließend noch in axialer Richtung geringfügig weiter eingetrieben werden, um die homogene Befüllung aller Zwischenräume mit Knochenzement, und einen guten Sitz des Oberschenkelteils zu sichern.

Besonders bevorzugt besitzen die Zweigbohrungen, in welche sich die Bohrung verzweigt, einen gegenüber der Bohrung reduzierten Durchmesser. Die Zweigbohrungen treten am Umfang verteilt aus dem Schaft aus. Besonders bevorzugt verläßt eine Zweigbohrung den Schaft lateral, und eine andere Zweigbohrung verläßt den Schaft medial, damit sich der flüssige Knochenzement leichter homogen um den Schaft der Prothese herum verteilen kann.

Besonders bevorzugt ist unterhalb des Stützkragens in dem Schaft eine umlaufende Umfangsnut eingeformt, in welcher der Distanzring lagert. Wenn dann der Distanzring abgezogen oder abgetrennt wird, so kann der Knochenzement - beim weiteren Nachpressen - auch die Umfangsnut füllen, wodurch der unmittelbare Kontakt zwischen dem metallischen Stützkragen und dem Patientenknochen noch besser verhindert wird.

Gemäß einer besonders bevorzugten Ausführungsform der Erfindung besitzt der Schaft medial eine Längsnut, welche in die Umfangsnut mündet. Wenn der Knochenzement beim Einpressen die mediale Längsnut erreicht, bildet der Knochenzement in dieser Längsnut eine rippenförmige Verdickung der Zementhülle. Diese Zementrippe bildet einen festen, formschlüssigen Verankerungssteg, welcher die Prothese insbesondere gegen eine Rotationsbeanspruchung oder Torsionsbeanspruchung um die Schaftachse sichert. Außerdem wird durch die mediale Längsnut der noch fließfähige Zement in Schaftrichtung gleichmäßiger verteilt, überschüssiger Zement kann insbesondere zum Stützkragen in die Umfangsnut abfließen.

Befindet sich auf dem Stützkragen ein konischer Zapfen zur Befestigung eines Prothesenkopfes, so verläuft die Bohrung bevorzugt zentral durch diesen Zapfen, und verläßt besonders bevorzugt den oberen Schaftabschnitt geradlinig in Verlängerung der Schaftachse. Auf diese Weise läßt sich ein ausreichender Querschnitt zum Durchpressen des Zementes verwirklichen, ohne daß der tragende Querschnitt des Oberschenkelteils dadurch zu stark geschwächt würde. Außerdem tritt der eingepreßte Zement ausreichend tief in den Knochenhohlraum ein, um gleichmäßig zum Schaftende und schließlich zum Stützkragen hin zu laufen. Bei Oberschenkelteilen mit einem lateralen nasenförmigen Ansatz am oberen Schaftende läßt sich die Bohrung alternativ auch - etwa parallel zur Zapfenachse - durch diesen Ansatz hindurchführen.

Gemäß einer besonders bevorzugten Ausführungsform der Erfindung besteht der Distanzring aus einem elastischen Dichtungsmaterial und zeigt in der Ebene unterhalb des Kragens eine solche Ausdehnung, daß er an seinem gesamten Umfang mit seiner Unterseite auf der Resektionskante des Patientenfemur aufliegt. Der Distanzring enthält bevorzugt ein oder mehrere Distanzelemente, die den gewünschten Abstand zwischen dem Schaft und der Knochenwand festlegen, wobei dieser Abstand eine Funktion des Umfangswinkels oder - alternativ - auch längs des gesamten Umfanges konstant sein kann. Bei dieser Ausführungsform der Erfindung läßt sich mit dem Einsetzen des Oberschenkelteils der zuvor entsprechend präparierte Knochenhohlraum durch den auf der Resektionskante aufliegenden Distanzring ausreichend luftdicht abdichten und dann durch eine durch die Knochenwand eingebrachte Saugöffnung vor und während des Einpressens des Zementes mit Unterdruck beaufschlagen, wodurch der Zement besonders rasch und gleichmäßig in den Zwischenraum zwischen Oberschenkelteil und Knochen eingezogen und verteilt wird. Nach dem Entfernen des Dichtringes muß dann zur endgültigen Positionierung das Oberschenkelteil noch geringfügig mechanisch tiefer in den Knochenhohlraum eingetrieben werden, wobei der nachquellende Zement die Umfangsnut und den Zwischenraum zwischen Stützkragen und Knochen füllt.

Der Distanzring besitzt bevorzugt eine Trennfuge oder eine Solltrennstelle und eine Zuglasche um Abziehen des Distanzringes, so daß sich das Distanzelement leicht manuell von dem Oberschenkelteil entfernen läßt.

Um die Verteilung des Knochenzementes während des Einpressens zu verbessern, und um außerdem die Rotationsfestigkeit und die Verankerung an dem Knochen zu verbessern, können in dem oberen und/oder im unteren Schaftbereich weitere Längsnuten in den Schaft eingeformt werden.

Vorteilhafte Weiterbildungen der Erfindung sind durch die Merkmale der Unteransprüche gekennzeichnet.

Im folgenden wird ein Ausführungsbeispiel der Erfindung anhand der Zeichnung näher erläutert.

Es zeigen:
- Fig. 1: eine Seitenansicht des Oberschenkelteils;
- Fig. 2: eine Vorderansicht des Oberschenkelteils der Fig. 1 in Richtung des Pfeiles II;
- Fig. 3 bis 7: Querschnitte längs der Linien III-III bis VII-VII der Fig. 2;
- Fig. 8: eine vergrößerte Darstellung des oberen Schaftbereiches des Oberschenkelteils gemäß Fig. 1;
- Fig. 9: eine der Fig. 8 entsprechende Darstellung im Schnitt;
- Fig. 10: eine Aufsicht auf den Distanzring; und
- Fig. 11: einen Querschnitt durch den Distanzring längs der Linie XI-XI der Fig. 10.
- Fig. 12: eine weitere Ausführungsform der Prothese in einer der Fig. 9 entsprechenden Darstellung.

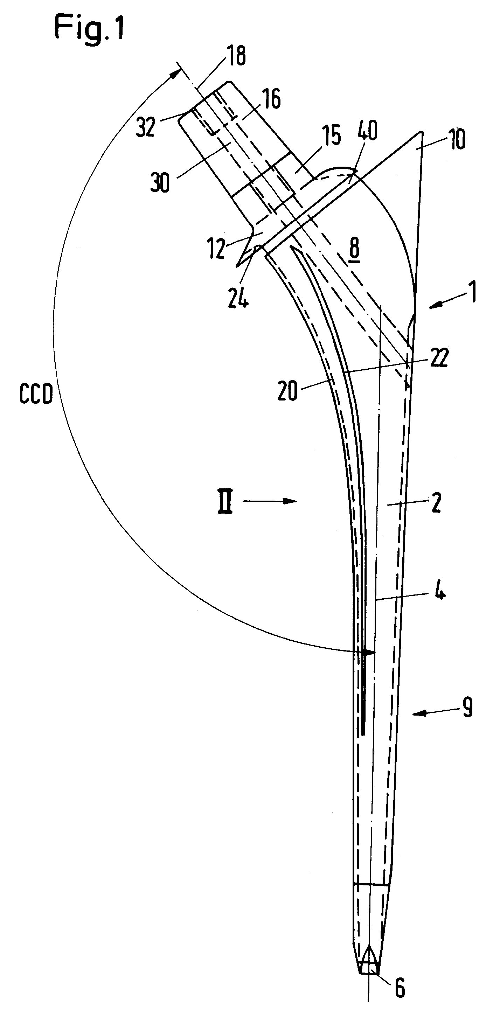

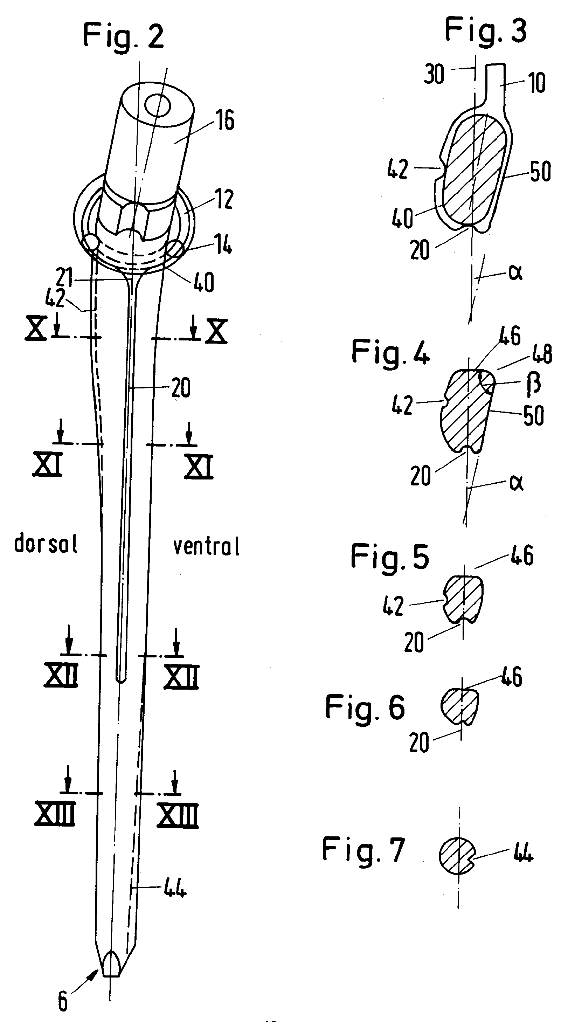

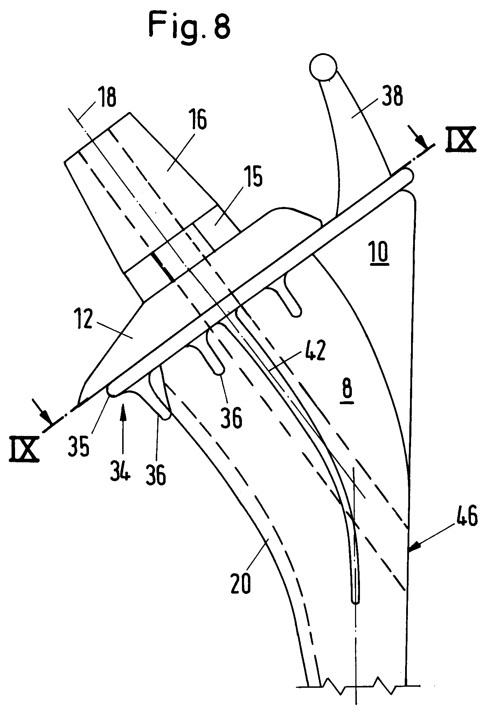

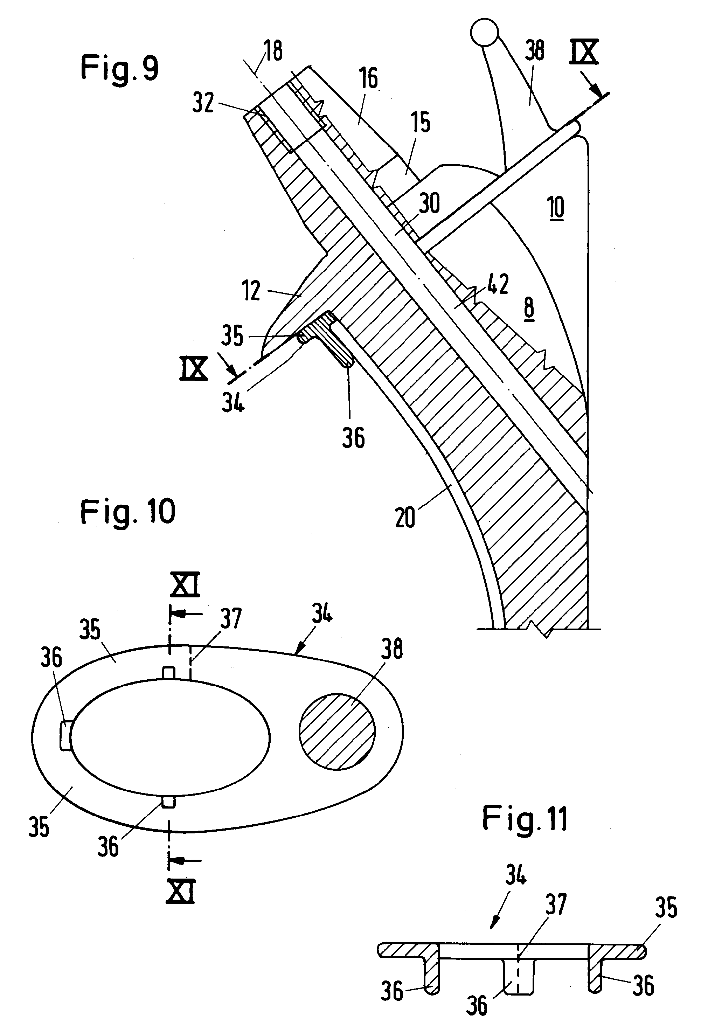

Die Fig. 1 und 2 zeigen ein einzementierbares Oberschenkelteil einer Hüftgelenk-Endoprothese von dorsal bzw. von medial. Die Fig. 8 und 9 zeigen eine der Fig. 1 entsprechende Teilansicht des oberen Endes des Oberschenkelteils in vergrößerter Darstellung, teilweise im Schnitt. Die Fig. 3 bis 7 stellen Querschnitte durch den Schaft 2 des Oberschenkelteils in verschiedenen Höhen dar. Das Oberschenkelteil 1 besitzt einen Schaft 2, der sich zu seinem unteren Ende 6 hin stetig verjüngt, wobei der Querschnitt des Schaftes näherungsweise jeweils dem Knochenhohlraum des natürlichen Femur angepaßt ist. Der Schaft trägt an seinem oberen, am sogenannten proximalen Ende 8 einen flanschartigen Stützkragen 12, der den Schaft 2 überragt. Auf dem Stützkragen 12 ist ein Mehrkantabschnitt 15 angeordnet, der in einen konischen Zapfen 16 zur Aufnahme eines prothetischen Hüftkopfes übergeht. Der Konusabschnitt 16, der Mehrkantabschnitt 15 und der obere Endabschnitt 8 des Schaftes 2 besitzen eine gemeinsame Achse 18, welche zur Schaftachse 4 einen vorgegebenen CCD-Winkel von beispielsweise 125° bis 160° einschließt. Statt der hier dargestellten einstükkigen Ausbildung von Schaft 2, Stützkragen 12 und konischen Zapfen 16 ist es alternativ auch möglich, den Schaft 2 nur mit einem Stützkragen 12 und einer längs der Achse 18 verlaufenden konischen Innenbohrung zu versehen, und den konischen Zapfen 16 als separates Teil auszubilden, der - in Richtung der Achse 18 - einen zusätzlichen Verankerungskonus enthält, welcher zur Verankerung in die konusförmige Innenbohrung des Schaftes 2 steckbar ist. Alternativ ist ebenfalls die Ausbildung des Oberschenkelteils mit einem einstückig angeformten prothetischen Hüftkopf möglich.

Der Querschnitt des Schaftes 2 ist - als Funktion des Abstands von seinem unteren Ende 6 - näherungsweise jeweils dem Querschnitt des Knochenhohlraums des natürlichen Femurknochen angepaßt. Er besitzt im unteren Bereich 9 des Schaftes 2 nahezu die Form eines Kreises und geht zum Stützkragen 12 hin in einen ovalen Querschnitt über. Unterhalb des Stützkragens 12 ist lateral am Schaft 2 ein Ansatz 10 angeformte der lateral nach außen verläuft und eine quer verlaufende Extraktionsbohrung 10a aufweist.

Der Schaft 2 besitzt in seinem medialen Bereich eine Längsnut 20, die sich im oberen Schaftabschnitt 8 vom Stützkragen 12 nach unten bis über die Mitte des Schaftes 2 hinaus erstreckt und im unteren Schaftabschnitt 9 ausläuft. Im oberen Schaftabschnitt 8 ist dorsal eine weitere Längsnut 42 eingeformt, welche unterhalb des Stützkragens 12 etwa in Richtung der Achse 18 des Zapfens 16 läuft und - der Schaftkrümmung folgend - schließlich in Richtung der Schaftachse 4 noch im oberen Schaftbereich 8 ausläuft. Ventral ist im unteren Schaftabschnitt 9 eine Längsnut 44 eingeformt, die unterhalb der Schaftmitte endet, vgl. die Fig. 2 und 7. Die Längsnuten 20, 42 und 44 nehmen bei der Implantation des Oberschenkelteils definiert Knochenzement in einer solchen Menge auf, die ausreicht, eine sichere formschlüssige Verankerung des Knochenzements im angrenzenden Knochengewebe sicherzustellen und - nach der Aushärtung - in den Längsnuten 20, 42, 44 Zementrippen zu bilden, welche eine Verdrehung des Oberschenkelteils 1 über die gesamte Schaftlänge, und damit eine Lockerung verhindert.

Die mediale Längsnut 20 und auch die dorsale Längsnut 42 weiten sich unterhalb des Stützkragens 12 auf und münden in eine Umfangsnut 40, die unterhalb des Stützkragens 12 in den Schaft 2 und gegebenenfalls auch noch eine vorgegebene Tiefe in den Stützkragen 12 eingeformt ist und dazu dient, daß beim Implantieren des Oberschenkelteils in den Längsnuten 20, 42 Zement gegen den Stützkragen 12 fließt und dort in einer zusammenhängenden Schicht auf der Unterseite des Stützkragens 12 verteilt wird, um einen unerwünschten direkten Kontakt zwischen dem Oberschenkelteil und dem Femurknochen auch im Bereich der Resektionskante des Knochens zu verhindern. Falls erwünscht, kann die Umfangsnut 40 zusätzlich mit einer Radialnut auf der Unterseite des Stützkragens 12 in Verbindung stehen, damit überschüssiger Zement während der Implantation nach außen weglaufen kann.

Wie sich insbesondere den Querschnitten gemäß den Fig. 3 bis 7 entnehmen läßt, ist lateral an den Schaft 2 im oberen Schaftabschnitt 8 eine ebene Anlagefläche 46 angeformt, die näherungsweise senkrecht zu der Medial-Lateral-Ebene 30 läuft und schließlich in den nasenförmigen Ansatz 10 übergeht, der im dargestellten Ausführungsbeispiel gegen die Medial-Lateral-Ebene 30 seitwärts versetzt ist. Um den Schaft 2 lateral im Patientenknochen verankern zu können, muß beim Implantieren eine entsprechende Ausnehmung in den Knochen eingearbeitet werden, die im lateralen Bereich der Prothese näherungsweise eine Formanpassung zwischen Prothese und Knochen darstellt. Der obere Schaftabschnitt 8 ist darüber hinaus so ausgebildet, daß dessen Querschnitt ventral näherungsweise von einer Geraden 50 begrenzt wird, welche die Medial-Lateral-Ebene 30 unter einem Winkel α schneidet, wobei sich der Winkel α über die Länge des Schaftes verändert. Die Anlagefläche 46 und der durch die jeweilige Gerade 50 definierte ventrale Oberflächenabschnitt bilden dabei - über eine vorgegebene Schaftlänge - eine abgerundete Kante 48, welche den Winkel β einschließt und - bei entsprechender Ausarbeitung des Knochenbettes - eine besonders wirksame Sicherheit gegen Verdrehung und Torsion bildet.

Wie insbesondere den Fig. 1 und 9 entnehmbar ist, besitzt das Oberschenkelteil 1 einen Distanzring 34 aus elastischem Material, der in der Umfangsnut 40 angeordnet ist und den Stützkragen 12 mindestens teilweise, und den Ansatz 10 vollständig flächig bedeckt und seitlich ausreichend überragt und während des Implantierens des Oberschenkelteils zu einem geeigneten Zeitpunkt entfernt werden kann. Zusätzlich zu der Deckfläche 35, welche die Unterfläche des Stützkragens 12 abdeckt und den Ansatz 10 überdeckt, besitzt der Distanzring 34 ein hierzu senkrecht verlaufendes, also sich in Richtung der Achse 18 an den Schaft anlegendes Distanzelement 36, das einen vorgegebenen Abstand zur Oberfläche des Schaftes 2 definiert. Die Dicke des Distanzelements 36 kann dabei über den gesamten Distanzring 34 konstant sein oder alternativ längs des Umfangs in gewünschter Weise variieren. Wie insbesondere der Fig. 10 entnehmbar ist, besitzt der Distanzring 34 eine Trennfuge 37, die ein einfaches Abziehen des Distanzringes 34, z.B. durch Zug an einer Zuglasche 38 gestattet, weil unter der Zugwirkung die Trennfuge 37 reißt.

Der Distanzring 34 erstreckt sich in der Ebene unterhalb des Stützkragens 12 an seinem gesamten Umfang auswärts bis an bzw. über die Resektionsfläche des Patientenfemur, um eine gute Abdichtung des Knochenhohlraumes zu verwirklichen. Außerdem kann sich der Stützkragen 12 bis über den lateralen Ansatz 10 erstrecken, der Distanzring verläuft dann unterhalb des Stützkragens 12 auch um den Ansatz 10 herum und wird bei Einsetzen der Prothese vom Stützkragen 12 an seinem gesamten Umfang gegen die Resektionsfläche des Patientenfemur gedrückt.

Die Dicke des Distanzelements 36 ist - ggf. durch zusätzlich angeformte Wulste oder Stege - so bemessen, daß das Distanzelement 36 beim Einsetzen des Oberschenkelteils in den Patientenfemur den Zwischenraum zwischen dem Oberschenkelteil und der harten Knochenwand des Patientenfemurs ausfüllt und dabei das Oberschenkelteil so im Patientenfemur positioniert, daß das Oberschenkelteil unterhalb des Stützkragens 12 an seinem gesamten Umfang von einem Freiraum gewünschter Stärke zur Aufnahme von Knochenzement umgeben ist.

Wie insbesondere den Fig. 1, 8 und 9 entnehmbar ist, ist vom oberen Ende des Oberschenkelteils 1 her eine Bohrung 30 eingearbeitet, die sich zentral durch den konischen Zapfen 16 erstreckt und in Verlängerung der Zapfenachse 18 verläuft und im oberen Schaftabschnitt 8 aus dem Oberschenkelteil 1 austritt. Die Bohrung 30 dient zum Einspritzen des Knochenzements nach dem Einsetzen des Oberschenkelteils 1 in den Knochenhohlraum. Um das Ansetzen der Spritzdüse der Zementspritze zu erleichtern, kann die Bohrung 30 an ihrem oberen Ende noch ein Innengewinde 32 o.dgl. zum lösbaren Ansetzen der Spritzdüse enthalten. Der Querschnitt der Bohrung 30 muß ausreichend groß sein, damit der Knochenzement in den Zwischenraum zwischen Knochenwandung und Oberschenkelteil hineinpreßbar ist. Andererseits soll ein zu großer Durchmesser der Bohrung 30 vermieden werden, um die ausreichende mechanische Stabilität des Oberschenkelteils zu gewährleisten.

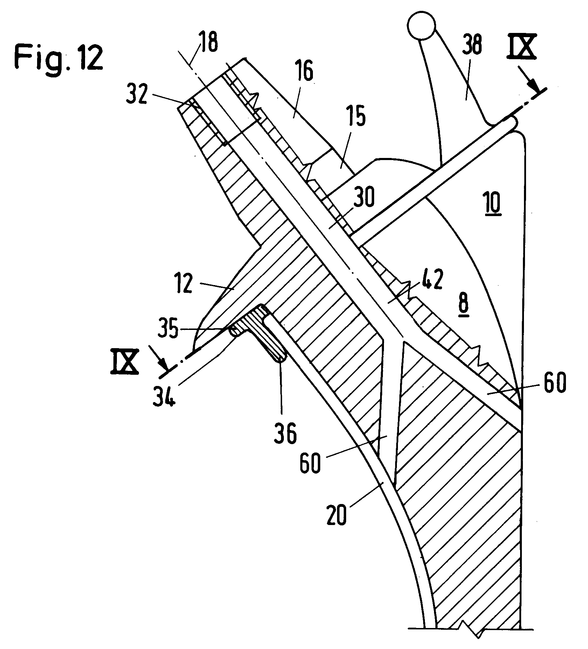

Fig. 12 zeigt eine weitere Ausführungsform des Oberschenkelteils einer Hüftgelenkprothese, die weitgehend derjenigen gemäß Fig. 9 entspricht. Während jedoch bei der Prothese gemäß Fig. 9 die Bohrung 30 mit konstantem Querschnitt durch den Zapfen 16, den Stützkragen 12 und den oberen Schaftabschnitt 8 hindurchläuft, ist die Bohrung 30 in der Ausführungsform gemäß Fig. 11 bis in den oberen Schaftabschnitt 8 hineingeführt und verzweigt sich dann dort in zwei Teilbohrungen 60. Eine Teilbohrung 60 ist mit reduziertem Querschnitt lateral aus dem Schaft 2 herausgeführt, die andere Teilbohrung 60 erstreckt sich - ebenfalls mit reduziertem Querschnitt - nach medial und tritt in der medialen Längsnut 20 aus dem Schaft 2 heraus. Zusätzlich können auch noch weitere Zweigbohrungen 60 am Umfang verteilt angeordnet und ggf. in der Höhe versetzt aus dem Schaft 2 austreten, um den Zement beim Einpressen gleichmäßiger um den Schaft zu verteilen.

Die Implantation des Oberschenkelteils 1 geschieht auf folgende Weise: Nach der Präparation des Knochenhohlraums wird das Oberschenkelteil zusammen mit dem Distanzring 34 in den Knochenhohlraum eingesetzt, so daß das Distanzelement 36 des Distanzringes 34 zwischen Knochenwand und Oberschenkelteil zu liegen kommt und die Lage des Oberschenkelteils im Knochenhohlraum festlegt. Außerdem drückt der Stützkragen 12 die Deckfläche 35 des Distanzringes 34 fest gegen die Resektionskante des Knochens, so daß der vom Distanzelement 36 definierte Hohlraum zwischen Knochenwand und Oberschenkelteil nach oben hin abgedichtet ist.

Anschließend wird der Knochenzement durch die Bohrung 30 in den Zwischenraum zwischen Oberschenkelteil 1 und Knochenwand eingepreßt. Durch ausreichenden Preßdruck ist sichergestellt, daß sich der Knochenzement gleichmäßig in diesem Zwischenraum verteilt und dadurch eine relativ homogene Zwischenschicht zwischen Knochenwand und Oberschenkelteil bildet. Die Längsnuten 20, 42 und 44 dienen dabei der Verteilung des Knochenzements in Längsrichtung des Schaftes 2. Ist der Zwischenraum zwischen Oberschenkelteil 1 und der Knochenwand mit Knochenzement gefüllt, so wird anschließend der Distanzring von der Prothese entfernt und weiterer Knochenzement nachgepreßt, damit ausreichend Knochenzement in den zuvor vom Distanzring angefüllten Raum unterhalb des Stützkragens gelangt und dadurch einen direkten Kontakt zwischen Stützkragen und Knochen verhindert. Falls erwünscht, kann an einer geeigneten Stelle auch noch eine Bohrung durch die Knochenwand von außen in den vorbereiteten Knochenhohlraum eingebracht werden und durch diese Bohrung der Knochenhohlraum mittels einer Saugpumpe während des Einpressens des Knochenzements unter Unterdruck gesetzt werden, wodurch die Einleitung des Knochenzements erheblich verbessert und beschleunigt wird.

## Patentansprüche

1. Einzementierbares Oberschenkelteil einer Hüftgelenk-Endoprothese,
mit einem sich zum unteren Ende hin verjüngenden Schaft,
und mit einem flanschartigen Stützkragen auf dem Schaft,
gekennzeichnet durch einen um den Schaft (2) umlaufenden, einen Abstand zwischen der Schaftoberfläche und der Knochenwand festlegenden, abtrennbaren elastischen Distanzring (34) an der Unterseite des Stützkragens (12), und eine von oben durch den Stützkragen (12) hindurchlaufende Bohrung (30), welche im oberen Schaftabschnitt (8) aus dem Oberschenkelteil (1) austritt.

2. Einzementierbares Oberschenkelteil einer Hüftgelenk-Endoprothese, mit einem sich zum unteren Ende hin verjügenden Schaft, und mit einem flanschartigen Stützkragen auf dem Schaft,
gekennzeichnet durch einen um den Schaft (2) umlaufenden, einen Abstand zwischen der Schaftoberfläche und der Knochenwand festlegenden, abtrennbaren elastischen Distanzring (34), der an der Unterseite des Stützkragens (12) anliegt, und eine von oben durch den Stützkragen (12) hindurchlaufende Bohrung (30), die sich im oberen Schaftabschnitt (8) in mehrere aus dem Schaft (2) austretende Zweigbohrungen (60) verzweigt.

3. Einzementierbares Oberschenkelteil nach Anspruch 2,
dadurch gekennzeichnet, daß die Zweigbohrungen (60) einen gegenüber der Bohrung (30) reduzierten Querschnitt besitzen.

4. Einzementierbares Oberschenkelteil nach Anspruch 2 oder 3,
dadurch gekenzeichnet, daß die Zweigbohrungen (60) am Umfang verteilt aus dem Schaft (2) austreten.

5. Einzementierbares Oberschenkelteil nach einem der Ansprüche 2 bis 4,
dadurch gekennzeichnet, daß eine Zweigbohrung (60) lateral und eine weitere Zweigbohrung (60) medial aus dem Schaft (2) austritt.

6. Einzementierbares Oberschenkelteil nach einem der vorstehenden Ansprüche,
dadurch gekennzeichnet, daß unterhalb des Stützkragens (12) in den Schaft (2) eine umlaufende Umfangsnut (40) eingeformt ist, und daß der Distanzring (34) in der Umfangsnut (40) angeordnet ist.

7. Einzementierbares Oberschenkelteil nach einem der vorstehenden Ansprüche,
dadurch gekennzeichnet, daß der Schaft (2) medial eine Längsnut (20) besitzt, die in die Umfangsnut (40) mündet.

8. Einzementierbares Oberschenkelteil nach einem der vorstehenden Ansprüche,
dadurch gekennzeichnet, daß die Bohrung (30) zentral durch einen auf dem Stützkragen (12) angeformten konischen Zapfen (16) verläuft.

9. Einzementierbares Oberschenkelteil nach Anspruch 8,
dadurch gekennzeichnet, daß die Bohrung (30) in Verlängerung der Zapfenachse (18) den oberen Schaftabschnitt (8) durchläuft.

10. Einzementierbares Oberschenkelteil nach einem der vorstehenden Ansprüche,
dadurch gekennzeichnet, daß am oberen Schaftende (8) lateral ein nasenförmiger Ansatz (10) angeformt ist, durch den die Bohrung (30) hindurchläuft, und daß sich der Distanzring (34) bis über den Ansatz (10) erstreckt.

11. Einzementierbares Oberschenkelteil nach einem der vorstehenden Ansprüche,
dadurch gekennzeichnet, daß der Distanzring (34) aus elastischem Dichtungsmaterial besteht.

12. Einzementierbares Oberschenkelteil nach einem der vorstehenden Ansprüche,
dadurch gekennzeichnet, daß der Distanzring (34) ein oder mehrere Distanzelemente (36) enthält, die einen vorgegebenen Abstand zu der Oberfläche des Schaftes (2) festlegen.

13. Einzementierbares Oberschenkelteil nach Anspruch 12,
dadurch gekennzeichnet, daß das bzw. die Distanzelemente (36) längs des Schaftumfanges einen unterschiedlichen Abstand zur Oberfläche des Schafts (2) festlegen.

14. Einzementierbares Oberschenkelteil nach einem der Ansprüche 12 oder 13,
dadurch gekennzeichnet, daß das Distanzelement (36) als an der Oberfläche des Schaftes (2) anliegender umlaufender Ansatz an dem Distanzring (34) angeformt ist.

15. Einzementierbares Oberschenkelteil nach Anspruch 12 oder 13,
dadurch gekennzeichnet, daß an dem Distanzring (34) mehrere, an der Oberfläche des Schafts (2) anliegende und in Schaftrichtung verlaufende Ansätze als Distanzelemente angeformt sind.

16. Einzementierbares Oberschenkelteil nach einem der vorstehenden Ansprüche,
dadurch gekennzeichnet, daß der Distanzring (34) eine Trennfuge (37) oder Solltrennstelle und eine Lasche (38) zum Abziehen des Distanzringes (34) enthält.

17. Einzementierbares Oberschenkelteil nach einem der vorstehenden Ansprüche,
dadurch gekennzeichnet, daß in dem oberen Schaftabschnitt (8) dorsal eine in die Umfangsnut (40) mündende Längsnut (42) eingeformt ist.

18. Einzementierbares Oberschenkelteil nach einem der vorstehenden Ansprüche,
dadurch gekennzeichnet, daß im unteren Schaftabschnitt (9) ventral eine Längsnut (44) eingeformt ist.

## Claims

1. The part of a hipjoint endoprosthesis which may be cemented into
the femur and has a shank which tapers in towards the lower end and a flangelike supporting collar on the shank,
characterized by a separable elastic spacer-ring (34) which runs round the shank (2) at the underside of the supporting collar (12) to establish a clearance between the surface of the shank and the wall of the bone, and by a bore (30) which runs through the supporting collar (12) from above and emerges from the femur-part (1) in the upper portion (8) of the shank.

2. The part of a hipjoint endoprosthesis which may be cemented into
the femur and has a shank which tapers in towards the lower end and a flangelike supporting collar on the shank,
characterized by a separable elastic spacer-ring (34) which runs round the shank (2) to establish a clearance between the surface of the shank and the wall of the bone and rests against the underside of the supporting collar (12), and by a bore (30) which runs through the supporting collar (12) from above and branches in the upper portion (8) of the shank into a number of branch bores (60) which emerge from the shank (2).

3. The part as in Claim 2,
of a hipjoint endoprosthesis which may be cemented into the femur, characterized in that the branch bores (60) have a cross-section which is reduced compared with the bore (30).

4. The part as in Claim 2 or 3,
of a hipjoint endoprosthesis which may be cemented into the femur, characterized in that the branch bores (60) emerge from the shank (2) distributed round the circumference.

5. The part as in one of the Claims 2 to 4,
of a hipjoint endoprosthesis which may be cemented into the femur, characterized in that one branch bore (60) emerges laterally from the shank (2) and a further branch bore (60) emerges medially.

6. The part as in one of the preceding Claims,
of a hipjoint endoprosthesis which may be cemented into the femur,
characterized in that a circumferential groove (40) is moulded into the shank (2), running round underneath the collar (12), and that the spacer-ring (34) is arranged in the circumferential groove (40).

7. The part as in one of the preceding Claims,
of a hipjoint endoprosthesis which may be cemented into the femur,
characterized in that the shank (2) has a longitudinal medial groove (20) which opens into the circumferentyial groove (40).

8. The part as in one of the preceding Claims,
of a hipjoint endoprosthesis which may be cemented into the femur,
characterized in that the bore (30) runs centrally through a conical neck (16) moulded onto the supporting collar (12).

9. The part as in one of the preceding Claims,
of a hipjoint endoprosthesis which may be cemented into the femur,
characterized in that the bore (30) runs through the upper portion (8) of the shank in prolongation of the axis (18) of the neck.

10. The part as in one of the preceding Claims,
of a hipjoint endoprosthesis which may be cemented into the femur,
characterized in that a lug (10) in the shape of a nose through which the bore (30) runs is moulded laterally onto the upper end (8) of the shank, and that the spacer-ring (34) extends right over the lug (10).

11. The part as in one of the preceding Claims,
of a hipjoint endoprosthesis which may be cemented into the femur,
characterized in that the spacer-ring (34) consist of elastic sealing material.

12. The part as in one of the preceding Claims,
of a hipjoint endoprosthesis which may be cemented into the femur,
characterized in that the spacer-ring (34) includes one or more spacer-elements (36) which establish a predetermined clearance to the surface of the shank (2).

13. The part as in Claim 12,
of a hipjoint endoprosthesis which may be cemented into the femur,
characterized in that the spacer-element or elements (36) establish along the circumference of the shank a varying clearance to the surface of the shank (2).

14. The part as in one of the Claims 12 or 13,
of a hipjoint endoprosthesis which may be cemented into the femur,
characterized in that the spacer-element (36) is moulded onto the spacer-ring (34) as a circumferential lug which rests against the surface of the shank (2).

15. The part as in Claim 12 or 13,
of a hipjoint endoprosthesis which may be cemented into the femur,
characterized in that a number of lugs running in the direction of the shank (2) and resting against its surface are moulded onto the spacer-ring (34) as spacer-elements.

16. The part as in one of the preceding Claims,
of a hipjoint endoprosthesis which may be cemented into the femur,
characterized in that the spacer-ring (34) includes a parting (37) or predetermined point of separation and a tongue (38) for pulling the spacer-ring (34) away.

17. The part as in one of the preceding Claims,
of a hipjoint endoprosthesis which may be cemented into the femur,
characterized in that a longitudinal groove (42) opening into the circumferential groove (40) is moulded dorsally into the upper portion (8) of the shank.

18. The part as in one of the preceding Claims,
of a hipjoint endoprosthesis which may be cemented into the femur,
characterized in that a longitudinal groove (44) is moulded ventrally into the lower portion (9) of the shank.

## Revendications

1. Partie fémorale, implantable avec ciment, d'une endoprothèse pour l'articulation de la hanche, comprenant une tige qui se rétrécit vers l'extrémité inférieure et un collet d'appui en forme de collerette prévu sur la tige, caractérisée par une bague d'écartement élastique séparable (34) qui entoure la tige (2), définit une distance entre la surface de la tige et la paroi de l'os et est appliquée contre la partie inférieure du collet d'appui (12), et par un perçage (30) qui traverse le collet d'appui (12) par le haut et débouche de la partie fémorale (1) dans le segment supérieur (8) de la tige.

2. Partie fémorale, implantable avec ciment, d'une endoprothèse pour l'articulation de la hanche, comprenant une tige qui se rétrécit vers l'extrémité inférieure et un collet d'appui en forme de collerette prévu sur la tige, caractérisée par une bague d'écartement élastique séparable (34) qui entoure la tige (2), définit une distance entre la surface de la tige et la paroi de l'os et est appliquée contre la partie inférieure du collet d'appui (12), et par un perçage (30) qui traverse le collet d'appui (12) par le haut et se ramifie, dans le segment supérieur (8) de la tige, en plusieurs perçages dérivés (60) débouchant de la tige (2).

3. Partie fémorale, implantable avec ciment, selon la revendication 2, caractérisée en ce que les perçages dérivés (60) possèdent un diamètre réduit par rapport au perçage (30).

4. Partie fémorale, implantable avec ciment, selon la revendication 2 ou 3, caractérisée en ce que les perçages dérivés (60) débouchent de la tige (2) en étant répartis sur sa périphérie.

5. Partie fémorale, implantable avec ciment, selon l'une des revendications 2 à 4, caractérisée en ce qu'un perçage dérivé (60) sort de la tige (2) en position latérale et un autre (60) en position médiale.

6. Partie fémorale, implantable avec ciment, selon l'une des revendications précédentes, caractérisée en ce qu'une gorge périphérique continue (40) est moulée dans la tige (2) sous le collet d'appui (12), la bague d'écartement (34) étant logée dans ladite gorge périphérique (40).

7. Partie fémorale, implantable avec ciment, selon l'une des revendications précédentes, caractérisée en ce que la tige (2) possède, en position médiale, une gorge longitudinale (20) qui débouche dans la gorge périphérique (40).

8. Partie fémorale, implantable avec ciment, selon l'une des revendications précédentes, caractérisée en ce que le perçage (30) traverse, en son centre, un tourillon conique (16) moulé sur le collet d'appui (12).

9. Partie fémorale, implantable avec ciment, caractérisée en ce que le perçage (30) traverse le segment supérieur (8) de la tige dans le prolongement de l'axe (18) du tourillon.

10. Partie fémorale, implantable avec ciment, selon l'une des revendications précédentes, caractérisée en ce que, à l'extrémité supérieure (8) de la tige est moulée, en position latérale, une partie rapportée (10) en forme de talon qui est traversée par le perçage (30), et en ce que la bague d'écartement (34) s'étend jusqu'au-dessus de la partie rapportée (10).

11. Partie fémorale, implantable avec ciment, selon l'une des revendications précédentes, caractérisée en ce que la bague d'écartement (34) est réalisée dans un matériau d'étanchéité élastique.

12. Partie fémorale, implantable avec ciment, selon l'une des revendications précédentes, caractérisée en ce que la bague d'écartement (34) comporte un ou plusieurs éléments d'écartement (36) qui définissent une distance prédéterminée par rapport à la surface de la tige (2).

13. Partie fémorale, implantable avec ciment, selon la revendication 12, caractérisée en ce que la ou les éléments d'écartement (36) définissent, le long de la périphérie de la tige, une distance variable par rapport à la surface de la tige (2).

14. Partie fémorale, implantable avec ciment, selon l'une des revendications 12 ou 13, caractérisée en ce que l'élément d'écartement (36) est moulé sur la bague d'écartement (34) sous la forme d'une partie rapportée circulaire appliquée contre la surface de la tige (2).

15. Partie fémorale, implantable avec ciment, selon la revendication 12 ou 13, caractérisée en ce que, sur la bague d'écartement (34), sont moulées plusieurs parties rapportées qui sont appliquées contre la surface de la tige (2), qui s'étendent dans le sens de la tige et qui font fonction d'éléments d'écartement.

16. Partie fémorale, implantable avec ciment, selon l'une des revendications précédentes, caractérisée en ce que la bague d'écartement (34) possède un joint de séparation (37) ou un point de séparation théorique et une patte (38) pour retirer la bague d'écartement (34).

17. Partie fémorale, implantable avec ciment, selon l'une des revendications précédentes, caractérisée en ce que, dans le segment supérieur (8) de la tige, est moulée, sur le côté dorsal, une gorge longitudinale (42) qui débouche dans la gorge périphérique (40).

18. Partie fémorale, implantable avec ciment, selon l'une des revendications précédentes, caractérisée en ce que, dans le segment inférieur (9) de la tige, sur le côté ventral, est moulée une gorge longitudinale (44).
